# EUROPÄISCHE PATENTANMELDUNG

(11) **EP 1 317 930 A1**
(43) Veröffentlichungstag der Anmeldung: **11.06.2003**
(21) Anmeldenummer: 02024787.0
(22) Anmeldetag: 07.11.2002
(51) Int. Cl.: A61K 38/57, A61P 9/00, A61P 37/06, A61P 43/00

(54) **Antithrombin zur Prävention und Therapie vaskuloproliferativer Erkrankungen**

(30) Priorität: 05.12.2001 DE 10159556
(71) Anmelder: Aventis Behring GmbH, 35002 Marburg (DE)
(72) Erfinder: Römisch, Jürgen, Dr., 2331 Vösendorf (AT); Stauss, Harald, 35232 Dautphetal (DE); Hölschermann, Hans, Dr., 35415 Pohlheim (DE)

(57) **Zusammenfassung**

Antithrombin, insbesondere Antithrombin III, ist ein geeignetes Mittel bzw. Wirkstoff zur Prävention und/oder Therapie von vaskuloproliferativen Erkrankungen wie z.B. Transplantatvaskulopathie, Restenose, In-Stent-Restenose und pulmonale Hypertonie.

## Beschreibung

Die vorliegende Erfindung betrifft die Verwendung von Antithrombin (AT), insbesondere die Verwendung von Antithrombin III (AT III), zur Prävention und/oder Therapie vaskuloproliferativer Erkrankungen. Weiterhin werden entsprechende Arzneimittel und Verfahren zu deren Herstellung von der vorliegenden Erfindung umfasst.

Unter vaskuloproliferativen Erkrankungen werden für die Zwecke der vorliegenden Erfindung bevorzugt Transplantatvaskulopathie, Restenose, In-Stent-Restenose und pulmonale Hypertonie verstanden. Aber auch die Prävention und/oder Therapie anderer Erkrankungen, bei denen eine für den Patienten schädliche Gefäßwandproliferation eine Rolle spielt, werden von der vorliegenden Erfindung umfasst.

Die Transplantatvaskulopathie (TVP) ist eine Form der koronaren Herzerkrankung, die das gesamte koronare Gefäßbett im transplantierten Herzen befällt. Sie kann zu raschen Gefäßverschlüssen führen und stellt die häufigste Todesursache herztransplantierter Patienten im ersten postoperativen Jahr dar. Ähnliche Gefäßveränderungen im koronaren Gefäßbett werden z. B. auch nach Ballonangioplastie und Stentimplantation sowie im pulmonalen Gefäßbett bei Patienten mit pulmonaler Hypertonie beobachtet.

Für die koronare Myointimaproliferation nach Herztransplantation (TVP) wird eine durch immunologische und nicht-immunologische Mechanismen hervorgerufene chronische endotheliale Schädigung verantwortlich gemacht, welche letztlich in einer Migration und Proliferation glatter Muskelzellen innerhalb des gesamten Koronargefäßsystems resultiert; vgl. Circulation 1997; 96: 2069. Trotz deutlicher Fortschritte auf den Gebieten der Immunsuppression, der Abstoßungsbehandlung, der Organkonservierung und der Beherrschung von kritischen Infektionen tritt die Transpantatvaskulopathie nach Transplantationen mit unverminderter Inzidenz und Ausprägung auf. Diese Tatsachen lassen bislang unbekannte kausale Faktoren in der Pathogenese dieser Erkrankung vermuten. Es existieren Hinweise, dass eine intravasale Hyperkoagulabilität mit auftretender Thrombenbildung und Fibrinablagerungen im Gefäßbett des transplantierten Herzens zusammen mit einer inflammatorisch-immunologischen Schädigung für die Intimaproliferation bei diesem Krankheitsbild eine Rolle spielen könnten. Bei herztransplantierten Patienten konnte z. B. eine latente Aktivierung des Hämostasesystems nachgewiesen werden; Circulation 1997; 96: 2302, Blood 2001; 97: 1079. Als mögliche Ursache einer solchen Gerinnungsaktivierung bei transplantierten Patienten kommen allerdings viele mögliche Faktoren und deren in dieser Beziehung noch unbekanntes Zusammenwirken in Betracht. So könnte z. B. eine lokale Gerinnungsaktivierung im Gefäßbett durch die Freisetzung einer Vielzahl von atherogenen Mediatoren wie z. B. Tissue Factor, Thrombin, Fibrin, Platelet Derived Growth Factor, basic-Fibroblast Growth Factor und andere, die die Migration und Proliferation von glatten Muskelzellen induzieren und damit für den Prozess der Myointimaproliferation bedeutsam sein können, erfolgen; Crit. Care Med. 2001;29:134, Int. Care Med. 2000; 26: 704, Circulation1997; 96: 4232.

Die Transplantatvaskulopathie ist jedoch kein herzspezifisches Problem der Transplantationsmedizin. Gleiche Gefäßwandproliferationen entwickeln sich auch nach anderen Organtransplantationen, insbesondere Lunge, Niere, Leber, Pankreas, und limitieren auch hier Tranplantatfunktion und- überleben. Die myointimale Proliferation scheint also eine immunologisch-inflammatorische Reaktion zu sein, mit der sich der Organismus mit einem Spenderorgan auseinandersetzt. Mit einer erfolgreichen Prävention und Therapie von TVP sollte also auch eine Prophylaxe der Transplantatabstoßung einhergehen.

Die im Zusammenhang mit der Transplantatvaskulopathie beschriebene schädliche Gefäßwandproliferation prägt auch das Krankheitsbild der primären und sekundären pulmonalen Hypertonie, und solche proliferativen Gefäßveränderungen werden auch bei Patienten bezüglich der Restenoseentstehung nach Ballonangioplastie bzw. Stentimplantation beobachtet.

Durch die Behandlung mit dem Antikoagulans Heparin wurde im Tierexperiment die Gefäßwandproliferation (Intimaproliferation) nach einer Herztransplantation allerdings nur partiell gehemmt: vgl. Circulation 1997; 96: 4232. Die Anwendung von Antithrombotika in den oben genannten Fällen zeigt offensichtlich nicht den gewünschten Effekt.

Somit stehen zur Zeit keine geeigneten protektiven oder therapeutischen Behandlungsmaßnahmen für die genannten vaskuloproliferativen Erkrankungen zur Verfügung. Aufgabe der vorliegenden Erfindung war es somit, für diese Fälle geeignete Behandlungsmaßnahmen bzw. pharmazeutische Mittel bereitzustellen.

Überraschenderweise wurde gefunden, dass Antithrombin, insbesondere Antithrombin III, zur Prävention und Therapie vaskuloproliferativer Erkrankungen, bevorzugt der genannten Erkrankungen, bzw. der damit verbundenen Intimaschädigung und Myointimaproliferation verwendet werden kann. Weiterhin umfasst die Erfindung die Verwendung von AT III zur Verhinderung einer Transplantatabstoßung durch den Empfängerorganismus.

Antithrombin III (AT III) ist ein Proteinase-Inhibitor, der neben Thrombin auch andere Proteasen wie Kallikrein und die Gerinnungsfaktoren des Blutplasmas IXa, Xa und Xlla hemmt. Man unterscheidet neben AT III noch weitere Formen von Antithrombin, z. B.

AT I - AT VI. Unter Antithrombin wird allgemein die gegen Thrombin gerichtete Aktivität des Blutplasmas verstanden.

Antithrombin/Antithrombin III sowie Verfahren zu seiner Gewinnung, z. B. aus Blutplasma, sind bekannt und beschrieben. Ferner ist Antithrombin III im Handel bzw. den Apotheken erhältlich.

Die vorliegende Erfindung umfasst somit auch Arzneimittel enthaltend Antithrombin und/oder Antithrombin III.

### Die vorliegende Erfindung wird durch das nachfolgende Beispiel näher erläutert:

Die antiproliferative Wirkung von AT III und damit seine Eignung zur Prävention und/oder Therapie vaskuloproliferativer Erkrankungen wurde tierexperimentell bei der heterotropen, allogenen Herztransplantation in der Ratte belegt. Hierbei handelt es sich um ein etabliertes Tiermodell zur Transplantatvaskulopathie, bei dem sich in den Transplantaten die gleichen Gefäßläsionen entwickeln wie nach humaner Herztransplantation. Die so gewonnen Ergebnisse ermöglichen direkte Aussagen in Bezug auf eine entsprechende Humananwendung.

Die transplantierten Ratten wurden in den ersten 14 Tagen nach der Transplantation mit Antithrombin III in einer Dosierung von 500 IU/kg Körpergewicht bzw. Placebo behandelt. Alle Tiere erhielten eine Standardimmunsuppression mit Cyclosporin. 120 Tage nach der Transplantation zeigten die AT-behandelten Tiere eine gegenüber der Placebogruppe signifikant geringe Ausprägung der Transplantatvaskulopathie. Die Ausprägung der koronaren Stenosierung wurde dabei in histologischen Schnittpräparaten der transplantierten Herzen nach einer HE/Elastica-Färbung ermittelt und anhand eines standardisierten Scoring-Systems in 4 Schweregrade graduiert. Dabei verringerte Antithrombin III sowohl die Inzidenz der Transplantatvaskulopathie (bei den AT-behandelten Transplantaten fanden sich deutlich mehr Gefäße ohne Intimaproliferation) als auch das Ausmaß der Gefäßwandproliferation (bei den AT-behandelten Transplantaten waren die Gefäße signifikant geringer stenosiert).

## Patentansprüche

1. Verwendung von Antithrombin, insbesondere Antithrombin III, zur Prävention und/oder Therapie vaskuloproliferativer Erkrankungen.

2. Verwendung nach Anspruch 1 zur Prävention und/oder Therapie der Transplantatvaskulopathie.

3. Verwendung nach Anspruch 1 zur Prävention und/oder Therapie der Restenose.

4. Verwendung nach Anspruch 1 zur Prävention und/oder Therapie der In-Stent-Restenose.

5. Verwendung nach Anspruch 1 zur Prävention und/oder Therapie der pulmonalen Hypertonie.

6. Arzneimittel enthaltend Antithrombin, insbesondere Antithrombin III.
